# EUROPEAN PATENT APPLICATION

(11) **EP 4 837 578 A1**
(43) Date of publication of application: **11.11.2026**
(21) Application number: 24926404.5
(22) Date of filing: 29.02.2024
(51) Int. Cl.: A61K 31/554, A61K 31/4178, A61P 1/08, A61P 9/06

(54) **SAFER DRUG AND USE THEREOF**

(71) Applicant: SHIJIAZHUANG ANTENG TECHNOLOGY CO., LTD, Shijiazhuang, Hebei 050035 (CN)
(72) Inventor: LI, Yingxin, Shijiazhuang, Hebei 050035 (CN); QI, Zhao, Shijiazhuang, Hebei 050035 (CN); WANG, Suping, Shijiazhuang, Hebei 050035 (CN)
(74) Representative: J A Kemp LLP
(86) International application number: PCT/CN2024/079252
(87) International publication number: WO 2025/179523

(57) **Abstract**

Provided are a safer drug and the use thereof. Provided are a pharmaceutical composition which is safer and which does not have toxicity leading to sudden cardiac death, and the use thereof. The provided safer drug is a compound formulation containing a 5-hydroxytryptamine receptor antagonist and diltiazem, and can effectively avoid the possibility of cardiac arrhythmia and sudden cardiac death due to ventricular depolarization and repolarization disorders caused by the 5-hydroxytryptamine receptor antagonist ondansetron. Further provided are the use of diltiazem **in** preparing a drug for improving electrical activity of the heart and a corresponding therapeutic method.

## Description

### TECHNICAL FIELD

The present application belongs to the field of western medicine, and specifically relates to a safer drug and use thereof, particularly to a novel compound formulation comprising ondansetron and diltiazem and use thereof, which can effectively avoid arrhythmia or sudden cardiac death induced by ondansetron.

### BACKGROUND

Ondansetron, with the chemical name 1,2,3,9-tetrahydro-9-methyl-3-[(2-methyl-1H-imidazol-1-yl)methyl]-4H-carbazol-4-one and the molecular formula C₁₈H₁₉N₃O, is a highly selective 5-hydroxytryptamine type 3 (5-HT₃) receptor antagonist. It exhibits high potency and high selectivity and can control vomiting induced by stimulation of receptors in the small intestine and the chemoreceptor trigger zone (CTZ). At the same time, ondansetron at antiemetic doses can enhance gastric emptying, helping to alleviate nausea; it also has anxiolytic effects on the central nervous system, which helps inhibit the excitation of the vomiting center. Ondansetron is currently a first-line antiemetic used both domestically and internationally, and is widely used clinically to prevent or treat nausea and vomiting induced by chemotherapy agents (such as cisplatin, doxorubicin, etc.), radiation therapy, surgery, or anesthesia.

The QT interval is a key part of the surface electrocardiogram, and cardiac electrical activity disorder is associated with a potentially fatal arrhythmia, Torsades de Pointes (TdP), and sudden cardiac death. For every 10 milliseconds (ms) increase in a patient's QT interval, the risk of progressing to TdP increases by 5%-7%. It is generally believed that when the absolute QT interval exceeds 500 ms, the risk increases significantly, and necessary interventions should be considered.

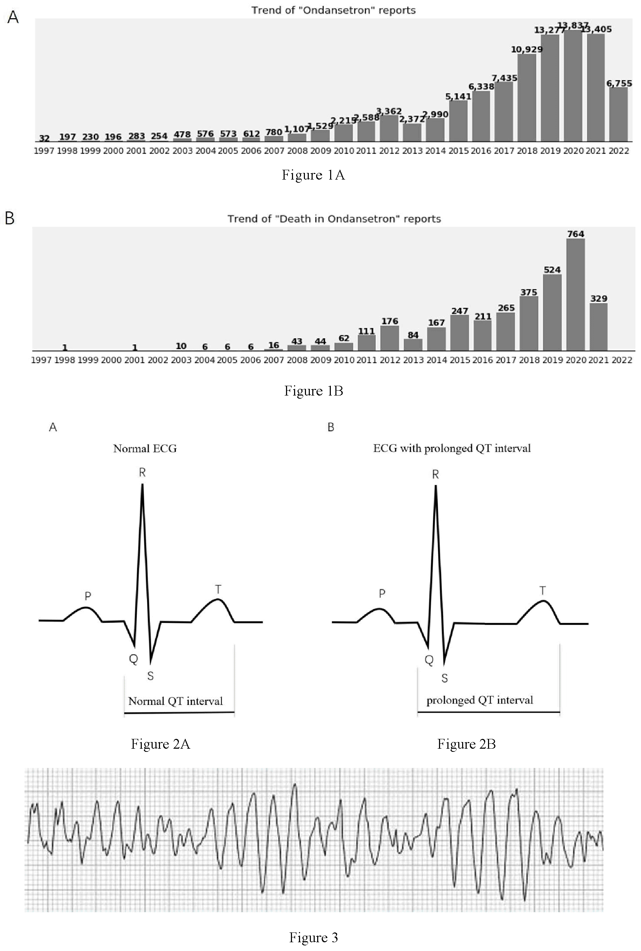

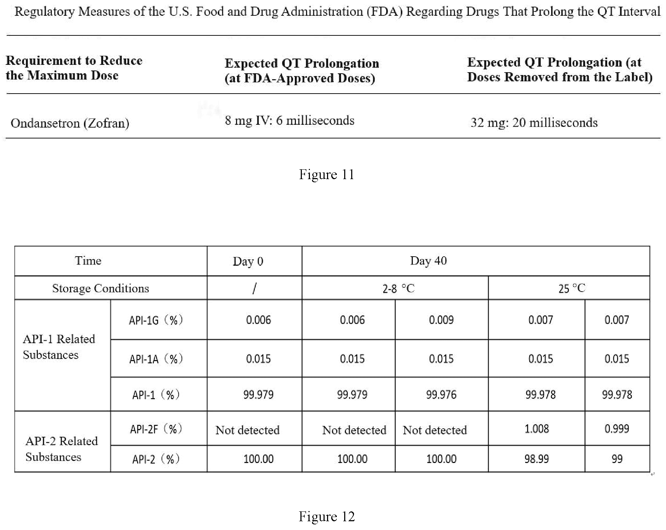

Transient electrocardiographic changes, including cardiac electrical activity disorders, have been reported in patients treated with ondansetron. In addition, post-marketing case reports have described instances of Torsades de Pointes in patients treated with ondansetron (the FDA issued a specific warning regarding this in 2012). A randomized, double-blind, cross-over clinical trial in the United States confirmed that the cardiac electrical activity disorders induced by ondansetron are dose-dependent. Specifically, following a single intravenous dose of 32 mg, the maximum mean difference in cardiac electrical activity disorders compared to placebo after baseline correction was 20 ms, whereas the maximum mean difference following a lower intravenous dose (8 mg) was 6 ms. Patients with high-risk factors of cardiac electrical activity disorder (such as congenital cardiac electrical activity disorder syndromes, congestive heart failure, bradyarrhythmias, or concurrent use of medications that may cause ventricular depolarization and repolarization disorders) are more susceptible to severe arrhythmias or even sudden death when using ondansetron. Therefore, it is generally recommended that a single intravenous dose of ondansetron should not exceed 16 mg. Companies such as GlaxoSmithKline have also revised the prescribing information for ondansetron to remove the 32 mg single intravenous dose. The following figures and tables (Figures 1 and 11) illustrate the adverse events associated with ondansetron and the regulatory measures taken by the FDA.

As shown in Figure 1, according to statistics from the eHealthMe website based on medical big data and artificial intelligence algorithms, as of July 19, 2022, 83,849 people had reported experiencing side effects while taking ondansetron, of which 3,448 (4.11%) died. Figure 1A shows the number of people reporting side effects from ondansetron each year; and Figure 1B shows the number of deaths among them each year.

### SUMMARY OF THE INVENTION

Although dose adjustment recommendations can reduce the probability of ondansetron-induced arrhythmia to a certain extent, they do not fundamentally eliminate this possibility. Patients receiving this medication, especially high-risk populations with congenital long QT syndrome or other underlying diseases, still have considerable risk.

To address the above problems, the applicant used a pluripotent stem cell cardiac electrophysiology research and development platform to design a new antiemetic drug that eliminates the cardiotoxic side effect associated with fatal arrhythmias, and discovered that diltiazem can effectively prevent ondansetron-induced cardiac electrical activity disorders, thereby reducing the possibility of arrhythmias, sudden cardiac death, and other issues.

In one aspect, the present application provides a novel safe antiemetic drug (improved novel drug) with reduced cardiac electrical activity disorder, comprising a 5-hydroxytryptamine receptor antagonist and diltiazem.

Further, the 5-hydroxytryptamine receptor antagonist is a 5-hydroxytryptamine type 3 receptor antagonist.

Further, the 5-hydroxytryptamine receptor antagonist is ondansetron.

Further, the novel safe drug is a compound formulation.

Further, the mass ratio of the 5-hydroxytryptamine receptor antagonist to diltiazem in the novel safe drug ranges from 180:1 to 1:4.

Further, the mass ratio of the 5-hydroxytryptamine receptor antagonist to diltiazem in the novel safe drug ranges from 35:1 to 5:1.

Further, the mass ratio of the 5-hydroxytryptamine receptor antagonist to diltiazem in the novel safe drug ranges from 24:1 to 12:1.

Further, the novel safe drug (improved novel drug) may be an oral formulation, an injectable formulation, a topical formulation, a spray, etc.; the oral formulation includes capsules, oral liquids, and tablets; the injectable formulation includes injections and powders for injection.

In another aspect, the present application provides the use of diltiazem in the manufacture of a drug for reducing cardiac electrical activity disorder.

Further, the cardiac electrical activity disorder is cardiac electrical activity disorder induced by a 5-hydroxytryptamine receptor antagonist.

Further, the 5-hydroxytryptamine receptor antagonist is a 5-hydroxytryptamine type 3 receptor antagonist.

Further, the 5-hydroxytryptamine receptor antagonist is ondansetron.

Further, the drug is an oral formulation, an injectable formulation, a topical formulation, a spray, etc.; the oral formulation includes capsules, oral liquids, and tablets; the injectable formulation includes injections and powders for injection.

Further, the administration dosage of diltiazem is 1-60 mg as a single oral dose or 0.0002-0.35 mg/kg for an injectable formulation.

Further, the administration dosage of diltiazem is 1-30 mg as a single oral dose or 0.0005-0.15 mg/kg for an injectable formulation.

Further, the plasma concentration of diltiazem ranges from 0.4 µg/L to 300 µg/L following administration of the compound formulation.

Further, the plasma concentration of diltiazem ranges from 2 µg/L to 130 µg/L following administration of the compound formulation.

Further, the plasma concentration of diltiazem ranges from 4 µg/L to 50 µg/L following administration of the compound formulation.

In another aspect, the present application provides a method for reducing cardiac electrical activity disorder, comprising administering diltiazem to a subject at risk of cardiac electrical activity disorder or having symptoms of cardiac electrical activity disorder.

Further, the subject is a human.

Further, the subject is a female.

Further, the cardiac electrical activity disorder is cardiac electrical activity disorder induced by a 5-hydroxytryptamine receptor antagonist.

Further, the 5-hydroxytryptamine receptor antagonist is a 5-hydroxytryptamine type 3 receptor antagonist.

Further, the 5-hydroxytryptamine receptor antagonist is ondansetron.

Further, the administration dosage of diltiazem is 1-60 mg as a single oral dose or 0.0002-0.35 mg/kg for an injectable formulation.

Further, the administration dosage of diltiazem is 1-30 mg as a single oral dose or 0.0005-0.15 mg/kg for an injectable formulation.

Further, the frequency and duration of administration of diltiazem range from 1 to 6 times per day for 1 to 14 consecutive days.

Further, the frequency and duration of administration of diltiazem range from 1 to 4 times per day for 1 to 7 consecutive days.

In another aspect, the present application relates to the use of diltiazem in the manufacture of a drug for reducing the risk of cardiac arrhythmia and sudden death induced by drugs.

Further, the cardiac arrhythmia and sudden death are cardiac electrical activity disorders induced by a 5-hydroxytryptamine receptor antagonist.

Further, the 5-hydroxytryptamine receptor antagonist is a 5-hydroxytryptamine type 3 receptor antagonist.

Further, the 5-hydroxytryptamine receptor antagonist is ondansetron.

Further, the drug is an oral formulation, an injectable formulation, a topical formulation, or a spray; the oral formulation includes capsules, oral liquids, and tablets; the injectable formulation includes injections and powders for injection.

Further, the administration dosage of diltiazem is 1-60 mg as a single oral dose or 0.0002-0.35 mg/kg for an injectable formulation.

Further, the administration dosage of diltiazem is 1-30 mg as a single oral dose or 0.0005-0.15 mg/kg for an injectable formulation.

In another aspect, the present application also relates to a method for reducing the risk of cardiac arrhythmia and sudden death, wherein the method comprises administering diltiazem to a subject at risk of cardiac electrical activity disorder or having symptoms of cardiac electrical activity disorder.

Further, the subject is a human.

Further, the subject is a female.

Further, the cardiac electrical activity disorder is cardiac electrical activity disorder induced by a 5-hydroxytryptamine receptor antagonist.

Further, the 5-hydroxytryptamine receptor antagonist is a 5-hydroxytryptamine type 3 receptor antagonist.

Further, the 5-hydroxytryptamine receptor antagonist is ondansetron.

Further, the administration dosage of diltiazem is 1-60 mg as a single oral dose or 0.0002-0.35 mg/kg for an injectable formulation.

Further, the administration dosage of diltiazem is 1-30 mg as a single oral dose or 0.0005-0.15 mg/kg for an injectable formulation.

Further, the frequency and duration of administration of diltiazem range from 1 to 6 times per day for 1 to 14 consecutive days.

Further, the frequency and duration of administration of diltiazem range from 1 to 4 times per day for 1 to 7 consecutive days.

Further, the plasma concentration of diltiazem ranges from 0.4 µg/L to 300 µg/L following administration of the compound formulation.

Further, the plasma concentration of diltiazem ranges from 2 µg/L to 130 µg/L following administration of the compound formulation.

Further, the plasma concentration of diltiazem ranges from 4 µg/L to 50 µg/L following administration of the compound formulation.

In the present application, the English name of ondansetron is Ondansetron, corresponding CAS number 99614-02-5, molecular formula C₁₈H₁₉N₃O; the English name of diltiazem is Diltiazem, corresponding CAS number 42399-41-7, molecular formula C₂₂H₂₆N₂O₄S; various numbers, names, and various hydrochlorides or other salts, other aliases, and trade names of commercial products, including but not limited to He xin shuang, He bao shuang, Tian er xin, De bao xing, etc., can be used interchangeably, representing the same drug.

In the present application, 5-hydroxytryptamine and 5-HT have the same meaning and can be used interchangeably.

In the present application, ondansetron and diltiazem may be formulated in a single pharmaceutical composition; they may also be provided as two separate drugs in the same package; or they may be packaged separately and used in combination; all forms of combination known and feasible in the art are applicable to the present application.

Dosage forms applicable in the present application include, but are not limited to, oral formulations and injectable formulations. The compound formulation, ondansetron formulation, and diltiazem formulation of the present application may be formulated in any clinically acceptable specific dosage form, including, but not limited to tablets, capsules, oral liquids, injections, powders for injection, topical formulations, sprays, etc. When ondansetron formulation and diltiazem formulation are provided in the same package or separately in various packages, their dosage forms may be the same or different.

Depending on the dosage form to be prepared or administered, various pharmaceutically acceptable excipients may be used, including but not limited to coating materials, solvents, solubilizers, binders, stabilizers, antioxidants, pH adjusters, flavoring agents. Those skilled in the art can select these excipient ingredients based on common pharmaceutical knowledge.

In addition to the above excipient ingredients, the formulation of the present application may also include other pharmaceutically acceptable excipients. The formulation of the present application may be provided alone, or when implementing the methods of the present application, other known antiemetic drugs or therapies may also be administered simultaneously, including but not limited to other 5-HT receptor antagonists, NK-1 receptor antagonists, dopamine receptor blockers, glucocorticoids, etc., such as granisetron, dolasetron, ramosetron, tropisetron, palonosetron, aprepitant, metoclopramide, dexamethasone, etc.; and other drugs or therapies for reducing cardiac electrical activity disorder, including but not limited to beta-blockers, sympathetic nerve blockers, such as propranolol, phenytoin sodium, reserpine, epinephrine, atropine, verapamil, sympathectomy, etc.

### Summary of the invention

### Beneficial Effects

1. It avoids ondansetron-induced cardiac electrical activity disorders, thereby reducing the possibility of arrhythmias, tachycardia, sudden cardiac death, and other issues.
2. It retains the effective antiemetic efficacy of ondansetron for prevention and treatment of vomiting.
3. For high-risk populations with congenital long QT syndrome or other underlying diseases, patients concurrently taking other drugs that cause cardiac electrical activity disorders, or patients with electrolyte imbalance (hypokalemia, hypocalcemia, hypomagnesemia), liver and kidney function decline, existing heart failure, left ventricular hypertrophy, myocardial infarction, and other heart diseases that create a predisposition to arrhythmias, the drug or method of the present application can be used more safely than ondansetron alone.
4. Diltiazem has a certain effect on improving myocardial blood supply and has a cardioprotective effect. The compound formulation containing diltiazem is more suitable for middle-aged and elderly people or patients with heart disease.
5. Females have a higher risk of drug-induced TdP than males, and the present application provides more significant protection for female patient populations.

### BRIEF DESCRIPTION OF THE DRAWINGS

Figure 1 shows statistics from the eHealthMe website based on medical big data and artificial intelligence algorithms. As of July 19, 2022, 83,849 people had reported experiencing side effects while taking ondansetron, of whom 3,448 (4.11%) died. Figure 1A shows the number of people reporting side effects from ondansetron each year; and Figure 1B shows the number of deaths among them each year.
Figure 2 is a schematic diagram of electrocardiograms (ECGs). Figure 2A shows a normal ECG of a healthy human individual. Figure 2B is a diagram of ECG with cardiac electrical activity disorder.
Figure 3 shows an ECG exhibiting Torsades de Pointes (TdP).

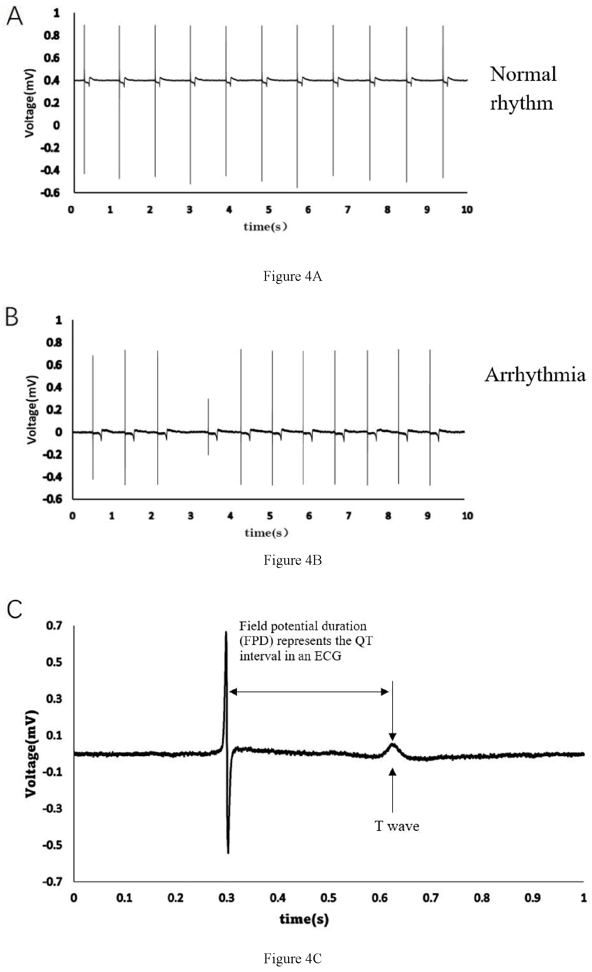

Figure 4 shows the field potential (FP) characteristics of human induced pluripotent stem cell-derived cardiomyocytes (hiPSC-CMs) recorded using a Multi-electrode Array (MEA). Figure 4A shows a representative normal MEA signal recording of the field potential electrical signals of hiPSC-CMs; Figure 4B shows a representative arrhythmic MEA signal recording of the field potential electrical signals of hiPSC-CMs, in which arrhythmic events can be detected; and Figure 4C demonstrates that MEA signal traces can be analyzed to obtain field potential parameters, including field potential duration (FPD), beating period, and corrected field potential duration (FPDc).

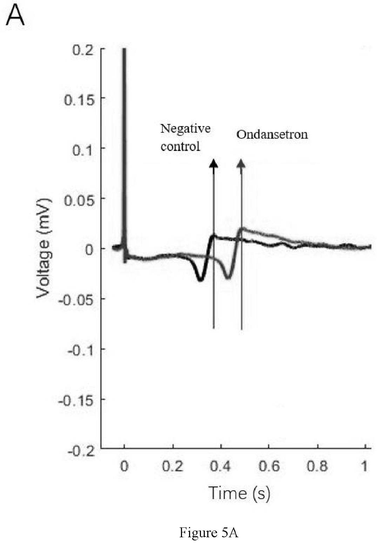

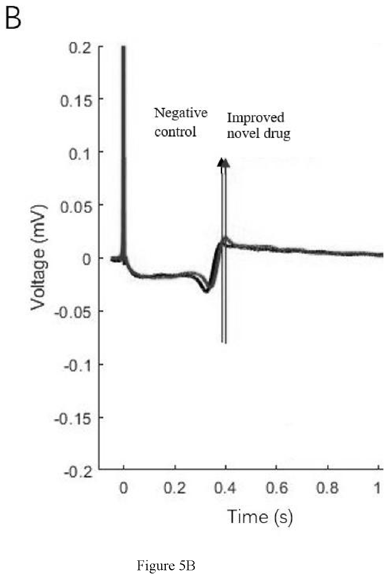

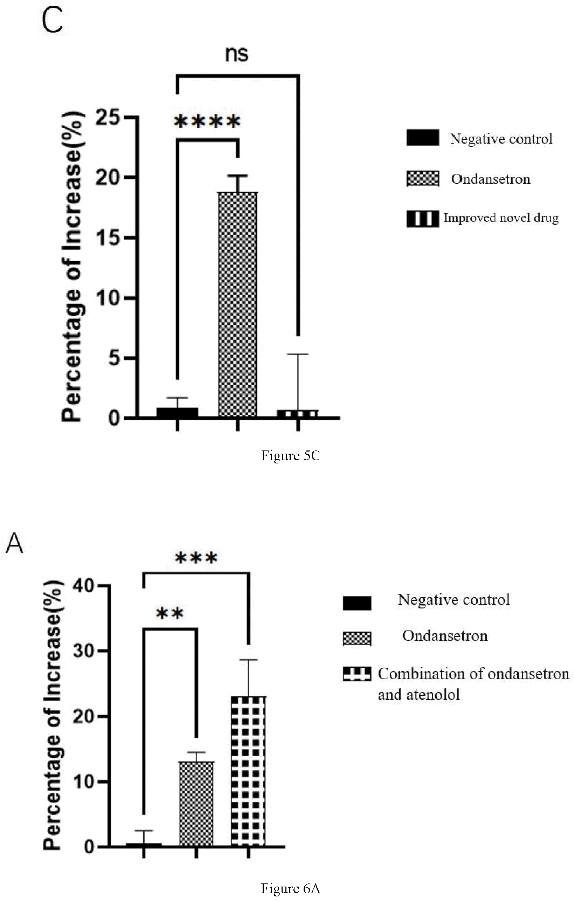

Figure 5 shows that diltiazem (improved novel drug group) can effectively reverse ondansetron-induced cardiac electrical activity disorder in cardiomyocytes, thereby preventing the development of related arrhythmias and avoiding sudden cardiac death caused by drug side effects. Figure 5A and Figure 5B show that ondansetron alone significantly increased FPDc compared to the control group, while the compound formulation of ondansetron and diltiazem (improved novel drug group) did not significantly change FPDc compared to the control group. Figure 5C shows raw field potential recordings demonstrating that ondansetron alone significantly increased FPDc compared to the control group, while the compound formulation of ondansetron and diltiazem (improved novel drug group) did not significantly change FPDc compared to the control group. FPDc (the rate corrected field potential duration) represents the heart rate-corrected field potential duration and is equivalent to the QT interval in an electrocardiogram. In this figure, the concentrations in the improved novel drug were 120 µg/L for ondansetron and 8.29 µg/L for diltiazem.

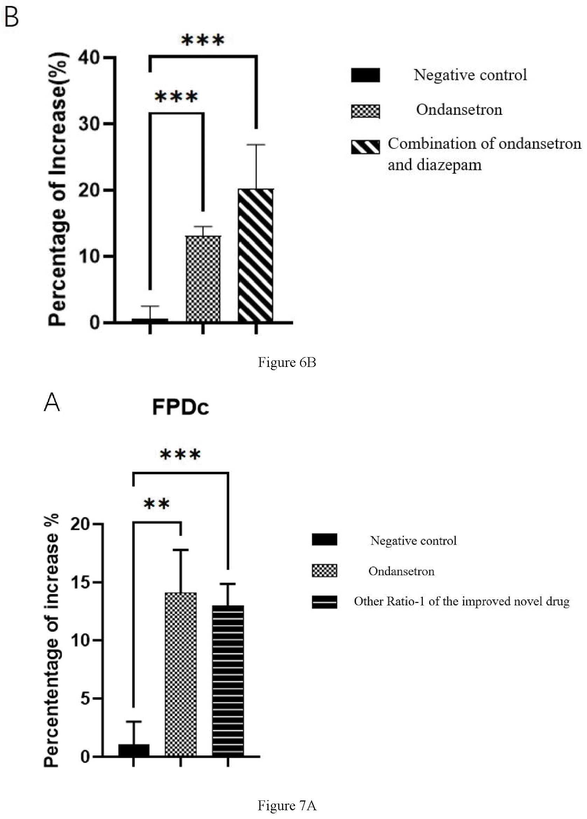

Figure 6 shows the effects of combining ondansetron with other drugs in the search for effective combinations. Figure 6A shows that atenolol could not reverse the toxicity of ondansetron. Figure 6B shows that diazoxide could not reverse the toxicity of ondansetron. In this figure, the concentration of ondansetron was 120 µg/L, the concentration of atenolol was 4.79 mg/L, and the concentration of diazoxide was 6.92 mg/L.

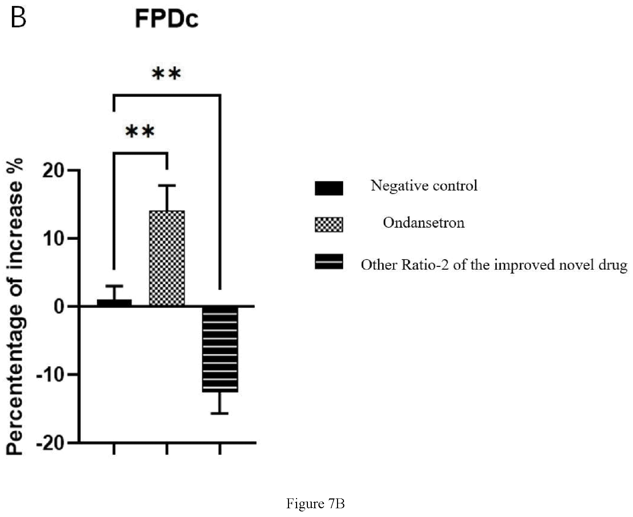

Figure 7 shows the effects of other concentrations combined with ondansetron during the search for effective combination concentrations. Figure 7A shows that in Other Ratio-1 of the improved novel drug, the concentration of diltiazem was too low to reverse the toxicity of ondansetron. Figure 7B shows that in Other Ratio-2 of the improved novel drug, the concentration of diltiazem was excessively high, leading to an excessive reversal of the toxicity of ondansetron. In this figure, the concentration of ondansetron was 120 µg/L, the concentration of diltiazem in Other Ratio-1 of the improved novel drug was 8.29 µg/L, and the concentration of diltiazem in Other Ratio-2 of the improved novel drug was 24.87 µg/L.

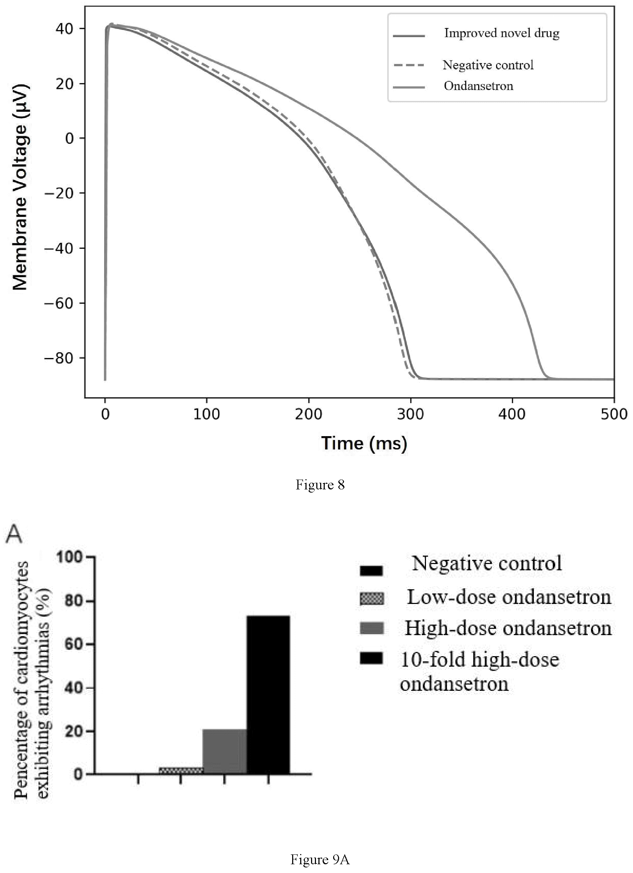

Figure 8 shows that in a computer-simulated in vitro proarrhythmia model, the action potential duration of the ondansetron group was longer than that of the negative control group. The improved novel drug group showed no significant difference from the negative control group. This indicates that the improved novel drug group can effectively avoid the alterations in myocardial action potentials induced by ondansetron.

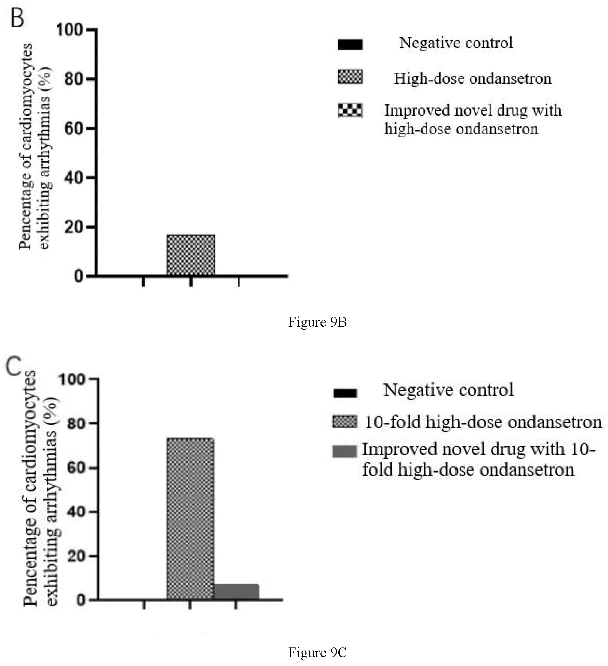

Figure 9 shows the incidence of arrhythmias in human cardiomyocytes under the influence of different drugs and concentrations. Figure 9A shows the percentage of cardiomyocytes exhibiting arrhythmias under the influence of different concentrations of ondansetron. Figure 9B shows the percentage of cardiomyocytes exhibiting arrhythmias induced by a high-dose group of ondansetron and its corresponding improved novel drug. Figure 9C shows the percentage of cardiomyocytes exhibiting arrhythmias induced by a 10-fold high-dose group of ondansetron and its corresponding improved novel drug.

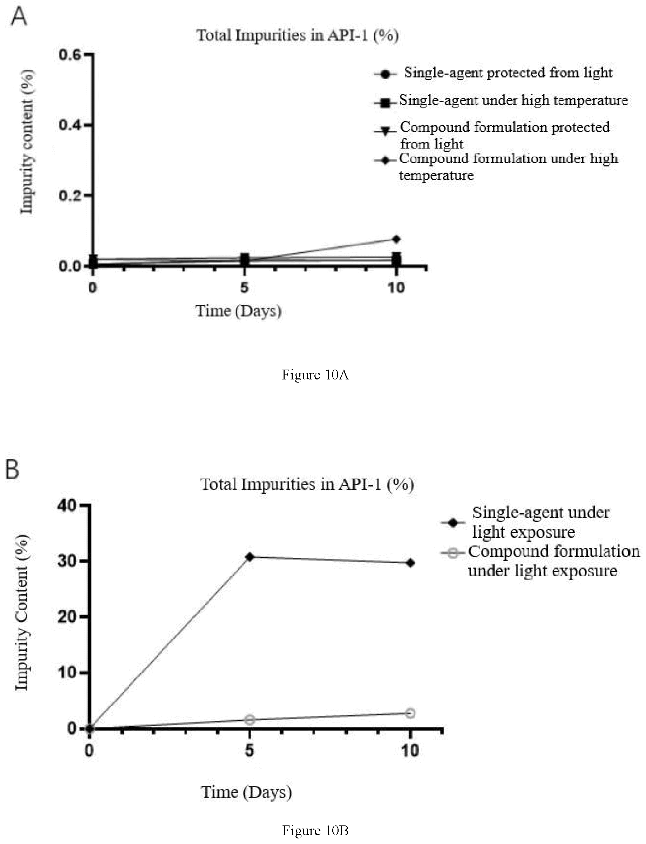

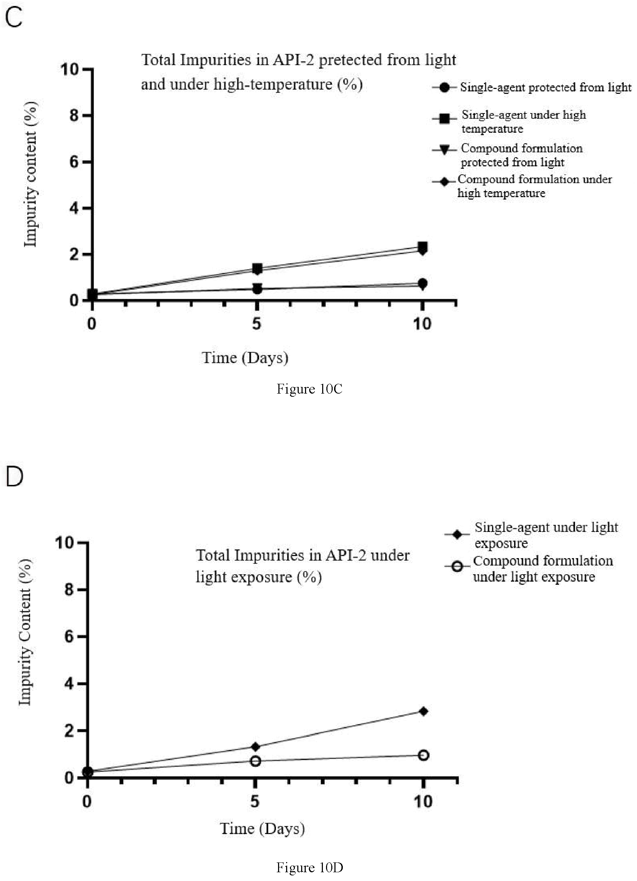

Figure 10 shows the effects of light protection, high temperature, and light exposure on the formulation of the improved novel drug and its components in influence factor tests. Herein, API-1 is ondansetron, and API-2 is diltiazem. Figure 10A shows that single API-1 and API-1 in the compound formulation are relatively stable under high temperature and light protection. Figure 10B shows that single API-1 is unstable under light exposure, while API-1 in the compound formulation is stable under light exposure, so single API-1 needs to be stored away from light. This indicates that under high temperature conditions, API-2 has no effect on the stability of API-1; under light exposure conditions, API-2 has a stabilizing effect on API-1, but there is still a significant degradation trend under light exposure, requiring storage protected from light. Figure 10C shows that single API-2 and API-2 in the compound formulation are unstable under high temperature but stable under light protection. Under high temperature and light protection conditions, API-1 has essentially no significant effect on the stability of API-2. Figure 10D shows that under light exposure conditions, API-2 in the compound formulation is more stable than single API-2. This indicates that API-2 is unstable under both high temperature and light exposure, and the compound formulation is unstable under both high temperature and light exposure, requiring storage at low temperature and protected from light.
Figure 11 shows the regulatory measures taken by the FDA for different doses of ondansetron.
Figure 12 shows the stability of API-1 (ondansetron) at 2-8°C and 25°C, with no significant changes observed in related substances. API-2 (diltiazem) remains relatively stable when stored at 2-8°C; however, under 25°C conditions, it primarily generates a degradation impurity, API-2F. Since API-2F is a metabolite formed in vivo, it exhibits high safety. It is predicted that the compound formulation will exhibit good stability when stored at low temperature (2-8°C) and protected from light, and its quality will meet requirements (no new safety issues will arise due to impurities).

### DETAILED DESCRIPTION

### Example 1: Drug Screening

ECGs of normal individuals, individuals with cardiac electrical activity disorder, and TdP are shown in Figures 2 and 3.

Using induced pluripotent stem cell technology integrating the latest international advancements in electrophysiology, pharmacology, and other high-tech fields, a pluripotent stem cell cardiac electrophysiology research and development platform focused on world-leading precision drug screening and development was established to design a new antiemetic drug that eliminates the cardiotoxic side effect of causing fatal arrhythmias. This platform can record and analyze parameters such as heart rate, QT, QTc, beating period, etc., and detect changes in these parameters induced by drugs.

A stem cell cardiac electrophysiology research and development platform was used to screen drug combination regimens. The basic procedure was as follows:
(1) Human induced pluripotent stem cell-derived cardiomyocytes were thawed and seeded onto Multi-electrode Array (MEA) cell culture plates, with the cell culture medium changed daily.
(2) After approximately 4 to 8 days, recording of cardiac cell electrical signals began. Baseline cardiac cell electrical signals prior to drug administration, as well as cardiac cell electrical signal data following the addition of drugs such as ondansetron and diltiazem, were recorded.
(3) Differences in electrical signals under the influence of different drugs were compared, the data were analyzed, and graphs for comparison were generated.

Figure 4 shows the field potential (FP) characteristics of human induced pluripotent stem cell-derived cardiomyocytes (hiPSC-CMs) recorded using a Multi-electrode Array (MEA).

### Example 2: Effect of the Compound Formulation of the Present Application

Human induced pluripotent stem cell-derived cardiomyocytes (hiPSC-CMs) were thawed and seeded onto a Multiple-electrode Array (MEA) plate pre-coated with hiPSC-CM coating solution. hiPSC-CM maintenance medium was used for medium changes, and thereafter the cell culture medium was changed daily until the completion of drug administration and recording. After 4-8 days of culture on the MEA plate, recording of cardiac electrical signals could begin. Baseline cardiac cell electrical signals were recorded prior to drug administration. Following the addition of ondansetron, cardiac electrical signal data were recorded. Subsequently, drugs such as diltiazem were added, and cardiac cell electrical signals were recorded. The recorded electrical signal data were analyzed, exported to Excel, and then plotted and analyzed using GraphPad.

Figure 5 shows that diltiazem (improved novel drug group) can effectively reverse ondansetron-induced cardiac electrical activity disorder in cardiomyocytes, thereby preventing the development of related arrhythmias and avoiding sudden cardiac death caused by drug side effects. Figure 5A and Figure 5B show that ondansetron alone significantly increased FPDc compared to the control group, while the compound formulation of ondansetron and diltiazem (improved novel drug group) did not significantly change FPDc compared to the control group. Figure 5C shows raw field potential recordings demonstrating that ondansetron alone significantly increased FPDc compared to the control group, while the compound formulation of ondansetron and diltiazem (improved novel drug group) did not significantly change FPDc compared to the control group. FPDc (the rate corrected field potential duration) represents the heart rate-corrected field potential duration and is equivalent to the QT interval in an electrocardiogram. In this figure, the concentrations in the improved novel drug were 120 µg/L for ondansetron and 8.12 µg/L for diltiazem.

Figure 6 shows the effects of several other drugs combined with ondansetron. Figure 6A shows that atenolol could not eliminate the toxicity of ondansetron. Figure 6B shows that diazoxide could not eliminate the toxicity of ondansetron. In this figure, the concentration of ondansetron was 120 µg/L, the concentration of atenolol was 4.79 mg/L, and the concentration of diazoxide was 6.92 mg/L.

Figure 7 shows the effects of other concentrations combined with ondansetron during the search for effective combination concentrations. Figure 7A shows that in Other Ratio-1 of the improved novel drug, the concentration of diltiazem was too low to reverse the toxicity of ondansetron. Figure 7B shows that in Other Ratio-2 of the improved novel drug, the concentration of diltiazem was excessively high, leading to an over-reversal of the toxicity of ondansetron. In this figure, the concentration of ondansetron was 120 µg/L, the concentration of diltiazem in Other Ratio-1 of the improved novel drug was 8.29 µg/L, and the concentration of diltiazem in Other Ratio-2 of the improved novel drug was 24.87 µg/L.

Figure 8 shows that in a computer-simulated in vitro proarrhythmia model, the action potential duration of the ondansetron group was longer than that of the negative control group. The improved novel drug group showed no significant difference from the negative control group. This indicates that the improved novel drug group can effectively avoid the alterations in myocardial action potentials induced by ondansetron.

Figure 9 shows the incidence of arrhythmias in human cardiomyocytes under the influence of different drugs and concentrations. Figure 9A shows the percentage of cardiomyocytes exhibiting arrhythmias under the influence of different concentrations of ondansetron. Figure 9B shows the percentage of cardiomyocytes exhibiting arrhythmias induced by a high-dose group of ondansetron and its corresponding improved novel drug. Figure 9C shows the percentage of cardiomyocytes exhibiting arrhythmias induced by a 10-fold high-dose group of ondansetron and its corresponding improved novel drug.

Figure 10 shows the effects of light protection, high temperature, and light exposure on the formulation of the improved novel drug and its components in influence factor tests. Herein, API-1 is ondansetron, and API-2 is diltiazem. Figure 10A shows that single API-1 and API-1 in the compound formulation are relatively stable under high temperature and light protection. Figure 10B shows that single API-1 is unstable under light exposure, while API-1 in the compound formulation is stable under light exposure, so single API-1 needs to be stored away from light. This indicates that under high temperature conditions, API-2 has no effect on the stability of API-1; under light exposure conditions, API-2 has a stabilizing effect on API-1, but there is still a significant degradation trend under light exposure, requiring storage protected from light. Figure 10C shows that single API-2 and API-2 in the compound formulation are unstable under high temperature but stable under light protection. Under high temperature and light protection conditions, API-1 has essentially no significant effect on the stability of API-2. Figure 10D shows that under light exposure conditions, API-2 in the compound formulation is more stable than single API-2. This indicates that API-2 is unstable under both high temperature and light exposure, and the compound formulation is unstable under both high temperature and light exposure, requiring storage at low temperature and protected from light.

Figure 11 shows the regulatory measures taken by the FDA for different doses of ondansetron.

Figure 12 shows the stability test results of the laboratory-optimized formulation.

## Claims

1. A safer drug, comprising a 5-hydroxytryptamine receptor antagonist and diltiazem.

2. The safer drug according to claim 1, wherein the 5-hydroxytryptamine receptor antagonist is a 5-hydroxytryptamine type 3 receptor antagonist.

3. The safer drug according to claim 2, wherein the 5-hydroxytryptamine receptor antagonist is ondansetron.

4. The safer drug according to any one of claims 1 to 3, wherein the safer drug is a compound formulation.

5. The safer drug according to claim 4, wherein the mass ratio of the 5-hydroxytryptamine receptor antagonist to diltiazem in the compound formulation ranges from 180:1 to 1:4; preferably from 35:1 to 5:1; more preferably from 24:1 to 12:1.

6. The safer drug according to any one of claims 1 to 5, wherein the compound formulation is an oral formulation, an injectable formulation, a topical formulation, or a spray; the oral formulation includes capsules, oral liquids, and tablets; the injectable formulation includes injections and powders for injection.

7. The safer drug according to claim 6, wherein the plasma concentration of diltiazem ranges from 0.4 µg/L to 300 µg/L following administration of the compound formulation.

8. The safer drug according to claim 7, wherein the plasma concentration of diltiazem ranges from 2 µg/L to 130 µg/L following administration of the compound formulation.

9. The safer drug according to claim 8, wherein the plasma concentration of diltiazem ranges from 4 µg/L to 50 µg/L following administration of the compound formulation.

10. Use of diltiazem in the manufacture of a drug for reducing the risk of drug-induced cardiac arrhythmia and sudden death.

11. The use according to claim 10, wherein the cardiac arrhythmia and sudden death are cardiac electrical activity disorders caused by a 5-hydroxytryptamine receptor antagonist.

12. The use according to claim 11, wherein the 5-hydroxytryptamine receptor antagonist is a 5-hydroxytryptamine type 3 receptor antagonist.

13. The use according to claim 12, wherein the 5-hydroxytryptamine receptor antagonist is ondansetron.

14. The use according to any one of claims 10 to 13, wherein the drug is an oral formulation, an injectable formulation, a topical formulation, or a spray; the oral formulation includes capsules, oral liquid, and tablets; the injectable formulation includes injections and powders for injection.

15. The use according to any one of claims 10 to 14, wherein the administration dosage of diltiazem is 1-60 mg as a single oral dose or 0.0002-0.35 mg/kg for an injectable formulation.

16. The use according to claim 15, wherein the administration dosage of diltiazem is 1-30 mg as a single oral dose or 0.0005-0.15 mg/kg for an injectable formulation.

17. A method for reducing the risk of cardiac arrhythmia and sudden death, wherein the method comprises administering diltiazem to a subject at risk of cardiac electrical activity disorder or having symptoms of cardiac electrical activity disorder.

18. The method according to claim 17, wherein the subject is a human.

19. The method according to claim 18, wherein the subject is a female.

20. The method according to any one of claims 17 to 19, wherein the cardiac electrical activity disorder is cardiac electrical activity disorder caused by a 5-hydroxytryptamine receptor antagonist.

21. The method according to claim 20, wherein the 5-hydroxytryptamine receptor antagonist is a 5-hydroxytryptamine type 3 receptor antagonist.

22. The method according to claim 21, wherein the 5-hydroxytryptamine receptor antagonist is ondansetron.

23. The method according to any one of claims 17 to 22, wherein the administration dosage of diltiazem is 1-60 mg as a single oral dose or 0.0002-0.35 mg/kg for an injectable formulation.

24. The method according to claim 23, wherein the administration dosage of diltiazem is 1-30 mg as a single oral dose or 0.0005-0.15 mg/kg for an injectable formulation.

25. The method according to any one of claims 17 to 22, wherein the frequency and duration of administration of diltiazem range from 1 to 6 times per day for 1 to 14 consecutive days.

26. The method according to claim 25, wherein the frequency and duration of administration of diltiazem range from 1 to 4 times per day for 1 to 7 consecutive days.

27. The method according to claim 17, wherein the plasma concentration of diltiazem after administration ranges from 0.4 µg/L to 300 µg/L.

28. The method according to claim 27, wherein the plasma concentration of diltiazem after administration ranges from 2 µg/L to 130 µg/L.

29. The method according to claim 28, wherein the plasma concentration of diltiazem after administration ranges from 4 µg/L to 50 µg/L.
